# FASCICULE DE BREVET EUROPEEN

(11) **EP 2 916 823 B1**
(45) Date de publication et mention de la délivrance du brevet: **15.02.2017**
(21) Numéro de dépôt: 13801640.7
(22) Date de dépôt: 07.11.2013
(51) Int. Cl.: A61K 9/14, A61K 31/57, A61P 15/00, A61P 15/18, A61K 47/32, A61K 9/20, A61K 9/48

(54) **PRODUIT DE CO-MICRONISATION COMPRENANT UN MODULATEUR SELECTIF DU RECEPTEUR A LA PROGESTERONE**
CO-MIKRONISATIONSPRODUCT ENTHALTEND EINEN SELEKTIVEN PROGESTERONREZEPTORMODULATOR
CO-MICRONISATION PRODUCT COMPRISING A PROGESTERONE RECEPTOR SELECTIVE MODULATOR

(30) Priorité: 08.11.2012 FR 1260605
(43) Date de publication de la demande: 16.09.2015
(73) Titulaire: Laboratoire HRA Pharma, 75003 Paris (FR)
(72) Inventeur: BATTUNG, Florian, F-75013 Paris (FR); JUVIN, Pierre-Yves, F-44100 Nantes (FR); HECQ, Jérôme, F-33360 Camblanes et Meynac (FR); COLIN, Aude, F-33100 Bordeaux (FR)
(74) Mandataire: Cabinet Becker et Associés
(86) Numéro de dépôt international: PCT/FR2013/052671
(87) Numéro de publication internationale: WO 2014/072647

(56) Documents cités:
- EP-A1- 0 803 250
- WO-A1-2009/134723
- WO-A2-2008/067086
- CN-A- 102 871 977
- US-A1- 2010 144 692
- GLASIER A F ET AL: "Ulipristal acetate versus levonorgestrel for emergency contraception: a randomised non-inferiority trial and meta-analysis", THE LANCET, LANCET LIMITED. LONDON, GB, vol. 375, no. 9714, 13 février 2010 (2010-02-13), pages 555-562, XP026904226, ISSN: 0140-6736, DOI: 10.1016/S0140-6736(10)60101-8 [extrait le 2010-01-29]
- AHMED S M ET AL: "Accelerated stability studies on 16-methylene-17alpha-acetoxy-19-nor-pregn- 4-ene-3,20-dione (Nestorone<TM>)", STEROIDS, ELSEVIER SCIENCE PUBLISHERS, NEW YORK, NY, US, vol. 60, no. 8, 1 août 1995 (1995-08-01), pages 534-539, XP004026506, ISSN: 0039-128X, DOI: 10.1016/0039-128X(95)00074-Z

## Description

### Domaine de l'invention

La présente invention concerne une nouvelle forme galénique d'un modulateur sélectif du récepteur à la progestérone (SPRM), plus précisément, un produit de co-micronisation, et des compositions pharmaceutiques contenant ladite forme galénique.

### Arrière-plan technologigue de l'invention

L'ulipristal acétate (en abrégé UPA) correspond à la 17α-acétoxy-11β-[4-N,N-diméthylamino-phényl)-19-norprégna- 4, 9-diène-3,20-dione (nomenclature IUPAC) et possède la formule chimique suivante :

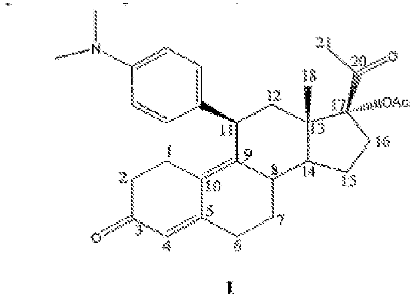

Sa synthèse est décrite, entre autres, dans le brevet EP 0 422 100 et dans la demande de brevet EP 1 602 662.

L'ulipristal acétate est un modulateur synthétique sélectif des récepteurs de la progestérone (SPRM). Par son action sur le récepteur de la progestérone, l'ulipristal acétate est capable d'exercer une action contraceptive en inhibant ou en retardant de l'ovulation. Des études cliniques ont montré que l'ulipristal acétate, administré en une dose unique de 30 mg, permet d'éviter une grossesse non désirée quand il est administré dans les 120 heures suivant un rapport sexuel non ou mal protégé (Glasier et al, Lancet. 2010, 375(9714):555-62 ; Fine et al, Obstet Gynecol. 2010, 115:257-63). L'ulipristal acétate a été ainsi autorisé en tant que contraceptif d'urgence et est commercialisé sous le nom commercial EllaOne® en Europe.

D'autres applications thérapeutiques de l'ulipristal acétate ont été proposées dans l'état de la technique. Des essais cliniques récents ont montré que l'administration chronique d'ulipristal acétate (à raison de 5mg ou 10 mg par jour) permet de réduire de manière significative les symptômes associés aux fibromes utérins et fournit un bénéfice thérapeutique supérieur à celui du traitement de référence, à savoir, l'acétate de leuprolide (Donnez et al., N Engl J Med. 2012; 366(5):421-32.). Sur la base de ces essais cliniques, l'Agence Européenne du Médicament (EMEA) a autorisé, en février 2012, la spécialité Esmya ® (5mg d'ulipristal acétate) pour le traitement pré-opératoire des symptômes associés aux fibromes utérins.

Les compositions pharmaceutiques actuellement commercialisées comportent de l'ulipristal acétate sous une forme micronisée.

La spécialité Esmya® se présente sous la forme d'un comprimé non-pelliculé comprenant 5 mg d'ulipristal acétate micronisé associés aux excipients suivants : cellulose microcristalline, mannitol, croscarmellose sodique, talc et stéarate de magnésium.

EllaOne® se présente, quant à elle, sous la forme d'un comprimé non-pelliculé comprenant 30 mg d'ulipristal acétate micronisé et les excipients suivants : lactose monohydraté, povidone K30, croscarmellose sodique et stéarate de magnésium.

Des compositions pharmaceutiques analogues ont été également décrites dans la demande internationale WO 2010/066749.

La mise au point de nouvelles formes galéniques adaptées pour l'administration des modulateurs sélectifs du récepteur à la progestérone tel que l'ulipristal acétate demeure un enjeu majeur pour leurs utilisations thérapeutique et contraceptive.

A cet égard, il existe, à l'heure actuelle, un besoin de nouvelles formulations pharmaceutiques contenant un modulateur sélectif du récepteur à la progestérone, tel que l'ulipristal acétate, et présentant des propriétés de libération et une biodisponibilité adaptées.

### Résumé de l'invention

La présente invention a pour objet un produit de co-micronisation comprenant :
- un principe actif choisi parmi le groupe constitué par les modulateurs sélectifs des récepteurs à la progestérone, leurs métabolites et leurs mélanges, et
- un excipient polymérique choisi parmi les polymères à base de N-vinyl-2-pyrrolidone et leurs mélanges.

Dans un mode de réalisation préféré, le principe actif est choisi parmi le groupe constitué par la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, l'ulipristal acétate et leurs mélanges.

Dans certains modes de réalisation, le produit de co-micronisation selon l'invention a une ou plusieurs des caractéristiques suivantes :
▪ le rapport massique « principe actif / excipient polymérique » est compris dans une gamme allant de 0,1 à 10, de préférence de 0,5 à 4,
▪ le principe actif est l'ulipristal acétate et l'excipient polymérique est choisi parmi le groupe constitué par une polyvinylpyrrolidone réticulée, une polyvinylpyrrolidone non-réticulée et leurs mélanges,
▪ le produit de co-micronisation peut comprendre, en outre, un surfactant solide, de préférence le dodécylsulfate de sodium,
▪ une d50 inférieure à 20 µm, de préférence inférieure à 15 µm, et/ou une d90 inférieure à 50 µm, de préférence inférieure à 40 µm.

La présente invention a également pour objet un procédé de préparation d'un produit de co-micronisation tel que défini précédemment, comprenant les étapes consistant à :
a) fournir un principe actif tel que défini ci-avant ;
b) mélanger le principe actif de l'étape a) avec un excipient polymérique tel que défini ci-avant et, en option avec un surfactant solide, de préférence le dodécylsulfate de sodium ; et
c) co-microniser le mélange obtenu à l'étape b).

Un objet supplémentaire selon l'invention est une composition pharmaceutique comprenant le produit de co-micronisation et un excipient acceptable sur le plan pharmaceutique. L'excipient acceptable sur le plan pharmaceutique peut être choisi parmi le groupe constitué par un diluant, un liant, un agent d'écoulement, un lubrifiant, un agent désintégrant et leurs mélanges.

Dans certains modes de réalisation, la composition pharmaceutique selon comprend:
- 0,5% à 80% de produit de co-micronisation,
- 15% à 95 % de diluant, et
- 0% à 5% de lubrifiant,
les pourcentages étant exprimés en poids par rapport au poids total de la composition.

La composition pharmaceutique selon l'invention peut comprendre de 1 mg à 100 mg, de préférence de 1 mg à 40 mg de principe actif par unité de dose. Elle peut être adaptée à une administration par voie orale et se présenter sous la forme d'une poudre, d'une granule, d'un comprimé pelliculé ou non pelliculé, ou d'une gélule.

La présente invention a également pour objet un produit de co-micronisation ou une composition pharmaceutique tels que définis précédemment pour une utilisation en tant que contraceptif par exemple, en tant que contraceptif régulier ou en tant que contraceptif d'urgence.

Enfin, l'invention a également pour objet un produit de co-micronisation ou une composition pharmaceutique, tels que définis précédemment, pour une utilisation dans le traitement ou la prévention d'un trouble gynécologique, de préférence affectant l'utérus.

### Figures

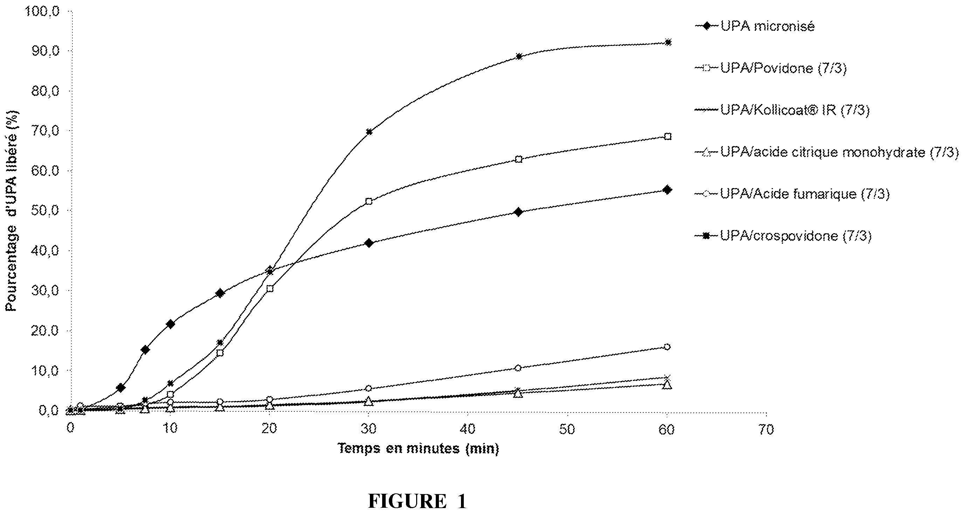

La **Figure 1** montre les courbes de dissolution *in vitro* pour différents co-micronisats (voir Exemple 1 ci-après) : UPA/povidone 7/3 (carré vide), UPA/crospovidone (carré plein), UPA/kollicoat® IR 7/3 (croix), UPA/acide citrique monohydrate 7/3 (losange vide), UPA/acide fumarique 7/3 (rond vide). Expérience témoin : UPA micronisé (seul - en absence d'excipient) losange plein). Ordonnées : pourcentage d'UPA libéré (%), abscisse : temps en minutes.

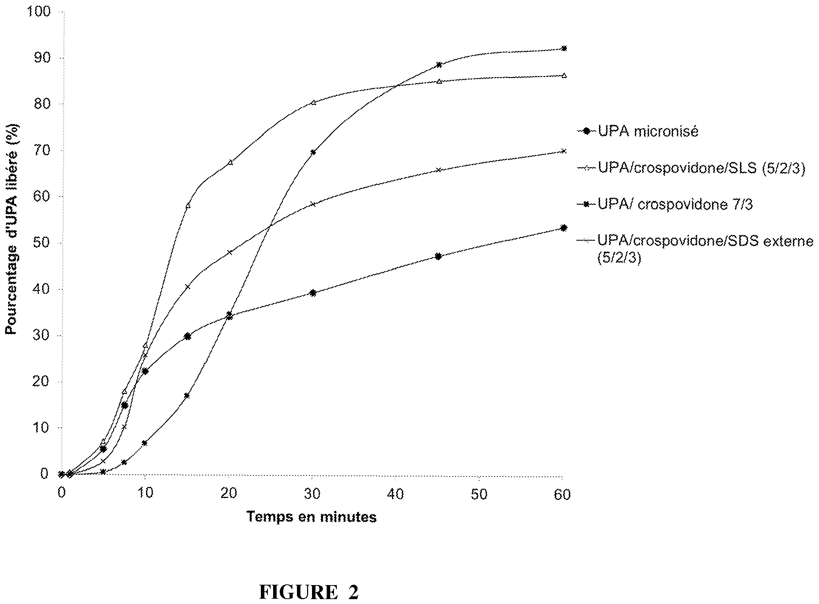

La **Figure 2** montre les courbes de dissolution *in vitro* pour différentes matrices de principe actif : co-micronisat UPA/crospovidone/SLS 5/2/3 (triangle vide), co-micronisat UPA/crospovidone 7/3 (carré plein), mélange comicronisat UPA/crospovidone + SLS (5/2/3) (croix). Expérience témoin : UPA micronisé (seul - en absence d'excipient - (rond plein)). Ordonnées : pourcentage d'UPA libéré (%), abscisse : temps en minutes.

### Description détaillée de l'invention

### Produit de co-micronisation selon l'invention et procédé de préparation

A l'issue de longues recherches, le Demandeur a montré qu'il est possible d'améliorer de manière significative le profil de dissolution *in vitro* de l'ulipristal acétate (ci-après UPA) grâce à une technologie de co-micronisation. Le Demandeur a montré que le produit résultant de la co-micronisation de l'ulipristal acétate avec un excipient polymérique de type polyvinylpyrrolidone présentait des propriétés de dissolution *in vitro* - en particulier un taux de dissolution à 45 minutes - significativement supérieures à celle de l'UPA micronisé seul, en l'absence d'excipient. Il est attendu que cette amélioration des propriétés de dissolution *in vitro* de l'UPA soit corrélée à une amélioration de la biodisponibilité *in vivo..* Par ses propriétés améliorées, le produit de co-micronisation pourrait permettre de diminuer les doses d'UPA à administrer au patient pour obtenir l'effet thérapeutique ou contraceptif recherché. La diminution de la dose d'UPA devrait permettre, entre autres, d'augmenter la sécurité, en particulier l'innocuité, des compositions pharmaceutiques finales. De manière remarquable, la co-micronisation de l'UPA ne conduit pas systématiquement à une amélioration de ses propriétés de dissolution. Les excipients polymériques de co-micronisation testés par le demandeur, autres que les polymères à base de N-vinylpyrrolidone, (voir exemple 1, tableau 1 ci-après), n'ont pas permis d'améliorer les propriétés de dissolution *in vitro* de l'UPA.

Par ailleurs, de manière inattendue, la co-micronisation de l'UPA avec un acide organique a conduit à une diminution nette de la vitesse de dissolution *in vitro* de l'UPA (voir Exemple 1, ci-après). Par ailleurs, le produit obtenu par co-micronisation de l'UPA avec un excipient polymérique de type de type PEG-PVA présente une vitesse de dissolution *in vitro* pour l'UPA très inférieure à celle observée pour l'UPA micronisée.

Ainsi, la présente invention a pour objet une nouvelle forme galénique, plus précisément, un produit de co-micronisation comprenant :
- un principe actif choisi parmi le groupe constitué par les modulateurs sélectifs des récepteurs à la progestérone, leurs métabolites et leurs mélanges, et
- un excipient polymérique à base de N-vinyl-2-pyrrolidone.

Par « produit de co-micronisation » (également désigné ci-après co-micronisat), on entend le produit obtenu par micronisation d'un mélange comprenant un principe actif et au moins un excipient.

On entend par « micronisation » un procédé permettant de réduire la taille des particules d'une poudre, par exemple, par broyage.

La réduction de la taille des particules est matérialisée par une diminution d'au moins 10% d'un paramètre choisi parmi la d50, la d10 et la d90. Une réduction « d'au moins 10% » englobe une réduction d'au moins 20%, d'au moins 30%, d'au moins 40%. La micronisation peut être effectuée au moyen de dispositifs disponibles dans le commerce, tels que les microniseurs à boules ou à jet d'air.

Dans le contexte de la présente invention, on entend par « un produit micronisé » un produit se présentant sous la forme d'une poudre ayant une d90 inférieure à 50 µm. Ainsi, de manière préférée, le produit de co-micronisation selon l'invention a une d90 inférieure à 50 µm.

On entend par un produit de co-micronisation ayant une d90 inférieure à 50 µm, un produit de co-micronisation, sous forme d'une poudre, dans lequel au moins 90% des particules a une taille inférieure à 50 µm.

On entend par un excipient polymérique à base de N-vinyl-2-pyrrolidone, ou encore un polymère à base de N-vinyl-2-pyrrolidone, un polymère comprenant la N-vinyl-2-pyrrolidone en tant que monomère. Un tel polymère englobe les homopolymères de N-vinyl-2-pyrrolidone et les copolymères de N-vinyl-2-pyrrolidone. L'excipient polymèrique peut être réticulé ou non-réticulé.

On entend par « copolymère» un polymère comprenant au moins deux types de monomères distincts. Il peut s'agir de copolymères dits aléatoires où les différents types de monomères s'enchainent de manière aléatoire ou encore des polymères dits « blocs ». Un exemple de copolymère à base de 1-vinyl-2-pyrrolidone est par exemple la copovidone qui est un copolymère de N-vinyl-2-pyrrolidone et d'acétate de vinyle. Dans certains modes de réalisation, l'excipient polymèrique est choisi parmi le groupe constitué par une polyvinylpyrrolidone réticulée (appelées ci-après crospovidone), une polyvinylpyrrolidone non-réticulée (appelées ci-après povidone), une copovidone et leurs mélanges

De préférence, l'excipient polymérique est choisi parmi le groupe constitué par une polyvinylpyrrolidone réticulée, une polyvinylpyrrolidone non-réticulée, une copovidone et leurs mélanges

Au sens de l'invention, une polyvinylpyrrolidone non-réticulée est composée de chaines polymériques libres, non liées entre elles par des liaisons covalentes.

Une polyvinylpyrrolidone réticulée est un réseau constitué de chaînes polymériques liées entre elles par des liaisons covalentes.

Les polyvinylpyrrolidones réticulées ou non-réticulées sont disponibles dans le commerce. On peut citer à titre d'exemple la crospovidone Polyplasdone® XL-10 commercialisée par ISP et la povidone Plasdone® K29-K32 commercialisée par BASF.

Dans certains modes de réalisation, l'excipient polymérique est choisi parmi les povidones ayant un poids moléculaire moyen allant de 10³ à 10⁷ g.mol⁻¹, de préférence allant de 3.10⁴ à 9.10⁴ g.mol⁻¹. Une povidone ayant un poids moléculaire moyen allant de 3.10⁴ à 9.10⁴ g.mol⁻¹ englobe un poids moléculaire moyen allant de 30000 à 40000 g.mol⁻¹, de 40000 à 50000 g.mol⁻¹, de 50000 à 60000 g.mol⁻¹, de 60000 à 70000 g.mol⁻¹, de 70000 à 80000 g.mol⁻¹et de 80000 à 90000 g.mol⁻¹. A titre d'exemple, un excipient polymérique adapté peut être une povidone ayant un poids moléculaire moyen allant de 55000 à 65000 g.mol⁻¹.

Dans d'autres modes de réalisation, l'excipient polymérique est choisi parmi le groupe constitué par les crospovidones.

On entend par « modulateur sélectif du récepteur de la progestérone » un ligand du récepteur à la progestérone qui exerce une activité agoniste, une activité antagoniste ou une activité mixte agoniste/antagoniste de manière tissu-spécifique, de préférence une activité agoniste ou agoniste/antagoniste mixte. Par ses connaissances générales, l'homme du métier pourra déterminer par des expériences de routine si un composé est un SPRM, en particulier en se référant aux articles de Smith et O'Malley, Endocrine Review, 25(1) :45-71, et de Chabbert-Buffet et al., Human Reproduction Update,2005, 11,293-307.

De manière préférée, le composé SPRM est un dérivé stéroïdien. Des exemples de SPRMs stéroidiens sont fournis dans les publications suivantes : Rao et al., Steroids, 1998, 63:523-530 et Chabbert-Buffet et al., Human Reproduction Update,2005, 11, 293-307. En particulier, Chabbert-Buffet et al. cite la mifépristone, l'onapristone, l'asoprisnil, l'ulipristal acétate, Org 33628 et Org 31710 comme étant des SPRM.

Les modulateurs spécifiques du récepteur à la progestérone sont de préférence des dérivés stéroïdiens substitués en position 11β par un groupe aryle.

Ainsi, dans un mode de réalisation avantageux, le ou les SPRM(s) présents dans le co-micronisat sont choisis parmi les composés de formule (I) suivante : 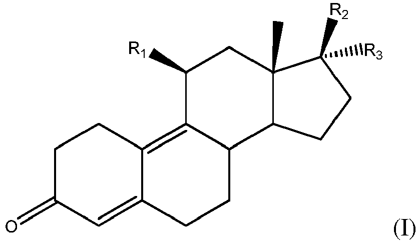 dans laquelle :
- R₁ représente un groupe aryle éventuellement substitué par un ou plusieurs groupes indépendamment en position ortho, para ou méta,
- R₂ représente -OH, un groupe alcoxy en C₁-C₅ ou -C(O)-R₄, et
- R₃ représente -OH, un groupe alcoxy en C₁-C₅, un alcynyle en C₂-C₅, un alcényle en C₂-C₅ ou -O-C(O)-R₅,
R₄ et R₅ étant choisis, indépendamment l'un de l'autre, parmi un groupe alkyle en C₁-C₃ et un groupe alcoxy en C₁-C₅,
ainsi que les sels pharmaceutiquement acceptables de ceux-ci.

Un alkyle en C₁-C₃ englobe les groupes méthyle, éthyle, propyle et isopropyle.

Un groupe alcoxy en C₁-C₅ englobe les groupes de formule -(CH₂)ₙO(CH₂)(_{y}-ₙ)CH₃, n étant un entier allant de 0 à 4, y étant un entier de 0 à 4 étant entendu que (y-n) est supérieur ou égal à 0.

Dans un mode préféré de réalisation, le SPRM est choisi parmi le groupe des composés SPRMs de formule (Ia) suivante:

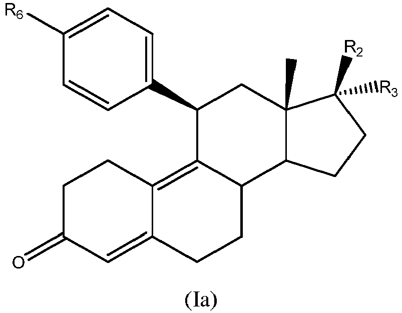

Dans laquelle :
- R₆ représente :
   - -NR₇R₈ dans lequel R₇ et R₈ représentent, indépendamment l'un de l'autre, -H ou un alkyle en C₁-C₃, R₇ et R₈ étant choisis de préférence parmi H et -CH₃ ;
   - -CH=N-O-R₉ dans lequel R₉ représente -H ou -C(O)-X-R₁₀ avec R₁₀ étant un alkyle en C₁-C₃ et X représentant O, NH ou S ; ou
   - -C(O)R₁₁ dans lequel R₁₁ représente un alkyle en C₁-C₃
- R₂ et R₃ étant tels que définis précédemment.

De tels composés comprennent, sans y être limités, la mifépristone, l'ulipristal acétate, l'asoprisnil et la télapristone. En particulier, la mifépristone correspond au composé de formule (Ia) dans laquelle R₆ est -N(CH₃)₂, R₂ est OH et R₃ est -C≡C-CH₃.

Dans un mode préféré de réalisation, le SPRM est choisi parmi le groupe des composés SPRMs de formule (Ia) dans laquelle :
- R₂ représente -OH, -OCH₃, -C(O)-CH₃ ou -C(O)-CH₂-O-CH₃
- R₃ représente -CH₂-O-CH₃, ou -O-C(O)-CH₃
- R₆ représente -NH₂, -NHCH₃, -N(CH₃)₂ ou -CH=N-OR₉ dans lequel R₉ représente H, -C(O)-S-C₂H₅ ou -C(O)-NH-C₂H₅.

Ce groupe de composés englobe, entre autres, la télapristone, l'asoprisnil, l'ulipristal acétate et certains de leurs métabolites.

Dans certains modes de réalisation, le SPRM est choisi parmi les composés de formule (Ia) dans laquelle R₆ est -CH=N-O-R₉. De tels composés englobent :
- Asoprisnil (R₉ est H, R₂ est OMe et R₃ est -CH₂OMe),
- J912 (R₉ est H, R₂ est H et R₃ est -CH₂OMe),
- J956 (également appelé Asoprisnil ecamate) (R₉ est -C(O)-NH-C₂H₅, R₂ est-OMe et R₃ est -CH₂OMe), et
- J1042 (R₉ est -C(O)-S-C₂H₅, R₂ est -OCH₃ et R₃ est -CH₂OMe).

Dans d'autres modes de réalisation, le SPRM est choisi parmi les composés de formule (Ia) dans laquelle R₆ est -NH₂, -NHCH₃, ou -N(CH₃)₂.

Dans un mode de réalisation préféré, le principe actif présent dans le co-micronisat est choisi parmi le groupe constitué par l'ulipristal acétate, les métabolites de l'ulipristal acétate et leurs mélanges.

Des métabolites de l'UPA sont décrits, entre autres, dans Attardi et al., Journal of Steroid Biochemistry and Molecular Biology, 2004, 88 : 277-288 et illustrés ci-après:

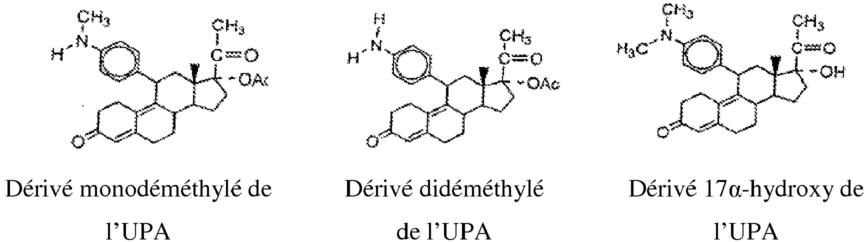

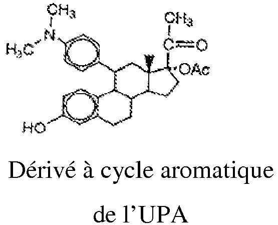

De préférence, le métabolite de l'ulipristal acétate est choisi parmi:
- la 17α-acétoxy-11β-[4-N-méthylamino-phényl)-19-norprégna-4,9-diène-3,20-dione (dérivé monodéméthylé) et
- la (17α-acétoxy-11β-[4-amino-phényl)-19-norprégna-4,9-diène-3,20-dione) (dérivé didéméthylé).

Dans un mode de réalisation préféré du co-micronisat selon l'invention, le principe actif est sélectionné parmi le groupe constitué par la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, l'ulipristal acétate et leurs mélanges.

Dans un autre mode préféré de réalisation, le produit de co-micronisation selon l'invention comprend :
▪ un principe actif choisi parmi le groupe constitué par l'ulipristal acétate, la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, et leurs mélanges, et
▪ un excipient polymérique choisi parmi le groupe constitué par les polyvinylpyrrolidones réticulées, les polyvinylpyrrolidones non-réticulées et leurs mélanges.

Dans un mode de réalisation supplémentaire, le produit de co-micronisation selon l'invention comprend :
▪ un principe actif, à savoir l'ulipristal acétate, et
▪ un excipient polymérique choisi parmi les polyvinylpyrrolidones réticulées et leurs mélanges.

A titre d'exemple, le produit de co-micronisation selon l'invention peut comprendre de l'ulipristal acétate en tant que principe actif et une crospovidone en tant qu'excipient polymérique.

Le rapport massique entre le principe actif et l'excipient polymérique dans le co-micronisat selon l'invention peut être compris dans une gamme allant de 0,1 à 10, de préférence de 0,5 à 4. Un rapport massique « principe actif/excipient polymérique » de 0,5 à 4 englobe un rapport massique de 0,5 à 1, de 1 à 1,5, de 1,5 à 2, de 2 à 2,5, de 3 à 3,5 et de 3,5 à 4. De préférence, le rapport massique « principe actif sur excipient polymérique » est compris dans une gamme allant de 1,5 à 4. Un rapport massique « principe actif/excipient polymérique » adapté est, par exemple, un rapport massique d'environ 2,3.

Dans certains modes de réalisation, le co-micronisat selon l'invention peut comprendre un excipient additionnel. Cet excipient additionnel peut permettre de potentialiser l'action de la povidone ou de la crospovidone sur les propriétés de dissolution de l'ulipristal acétate. Cet excipient peut être choisi parmi les surfactants communément utilisés en galénique et pouvant subir une co-micronisation, typiquement par broyage. On entend par « surfactant solide » un surfactant qui est solide à température ambiante, c'est-à-dire typiquement à environ 20°C. Dans certains modes de réalisation avantageux, le surfactant présente une température de fusion élevée, de préférence supérieure à 50°C et de manière encore plus préférée supérieure à 100°C. Le surfactant peut être choisi parmi les sels d'alkylsulfates en C₈-C₂₀, de préférence en C₁₀-C₁₄ et leurs mélanges.

Dans un mode de réalisation avantageux, le surfactant est choisi parmi les sels de dodécylsulfate, de préférence, les sels alcalins ou alcalino-terreux de celui-ci tel qu'un sel de sodium, de magnésium ou de calcium. Un surfactant particulièrement adapté pour l'obtention d'un produit de co-micronisation selon l'invention est le SDS c'est-à-dire le dodécyl-sulfate de sodium.

Ainsi, dans un mode de réalisation préféré, le surfactant est le dodécyl-sulfate de sodium.

Dans un mode de réalisation particulier, le produit de co-micronisation comprend :
▪ un principe actif choisi parmi le groupe constitué par l'ulipristal acétate, la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, et leurs mélanges, de préférence l'ulipristal acétate,
▪ un excipient polymérique choisi parmi les polyvinylpyrrolidones réticulées, les polyvinylpyrrolidones non-réticulées et leurs mélanges, et
▪ un surfactant solide, de préférence le SDS.

Le rapport massique entre le principe actif et le surfactant est généralement compris dans une gamme allant de 0,1 à 10, de préférence, de 0,5 à 4. Un rapport massique « principe actif/surfactant» adapté est, par exemple, un rapport massique d'environ 1,7. A titre d'exemple, un co-micronisat selon l'invention peut comprendre un principe actif, un excipient polymérique et un surfactant en des quantités vérifiant les ratios massiques suivant :
▪ un rapport massique « principe actif/surfactant » de 1 à 2, de préférence environ 1,7 et
▪ un rapport massique « principe actif/excipient polymérique » de 2 à 4, de préférence d'environ 2,5.

La granulométrie (c'est-à-dire la distribution de la taille des particules) du produit de co-micronisation peut avoir une incidence sur les propriétés de solubilité de l'UPA.

Il est préférable que la d50 du produit de co-micronisation soit inférieure à 25 µm, de préférence inférieure à 20 µm, voire inférieure à 15 µm.

Une d50 inférieure à 15 µm englobe une d50 inférieure à 12 µm, à 11 µm, à 10 µm, à 9 µm, à 8 µm, à 7 µm, à 6 µm, à 5 µm, 4 µm.

Il est également préférable que la d90 du produit de co-micronisation soit inférieure à 50 µm, voire inférieure à 40 µm.

Une d90 inférieure à 40 µm englobe une d90 inférieure à 38 µm, à 37 µm, à 36 µm, à 35 µm, à 34 µm, à 33 µm, à 32 µm, à 31 µm, à 30 µm, à 29 µm, à 28 µm, à 27 µm, à 26 µm, à 25 µm, à 24 µm, à 23 µm, à 22 µm, à 21 µm, à 20 µm, à 19 µm, à 18 µm, à 17 µm, à 16 µm, à 15 µm, à 14 µm, à 13 µm, à 12 µm, à 11 µm, à 10 µm.

Dans certains modes de réalisation, le produit de co-micronisation selon l'invention est caractérisé en ce que sa granulométrie présente :
- une d50 inférieure à 20 µm, de préférence inférieure à 15 µm, et/ou
- une d90 inférieure à 50 µm, de préférence inférieure à 40 µm et de manière encore plus préférée inférieure à 30 µm.

A titre d'exemple, le co-micronisat selon l'invention peut avoir une d50 inférieure à 5µm et/ou une d90 inférieure à 15 µm.

La d10 du co-micronisat selon l'invention est généralement supérieure à 0,05 µm.

Dans le contexte de la présente invention, « une d50 inférieure à X µm » signifie qu'au moins 50% des particules du co-micronisat ont une taille inférieure à X µm.

« une d90 inférieure à Y µm» signifie qu'au moins 90% des particules du co-micronisat ont une taille inférieure à Y µm.

De même, « une d10 supérieure à Z µm » signifie qu'au moins 90% des particules du co-micronisat ont une taille de particule supérieure à Z µm.

La granulométrie - c'est-à-dire la distribution de la taille des particules - du produit de co-micronisation - et en particulier les paramètres d90, d50 et d10 - peuvent être déterminés par toute méthode connue de l'homme du métier. De manière préférée, on utilisera la diffraction laser.

Le produit de co-micronisation présente des propriétés améliorées de dissolution *in vitro* du principe actif. Dans certains modes de réalisation, le produit de co-micronisation selon l'invention est caractérisé en ce qu'au moins 55% du principe actif qu'il contient est libéré en 45 minutes lorsque ledit produit de co-micronisation est soumis à un test de dissolution *in vitro,* de préférence selon la Pharmacopée Européenne §2.9.3.

Le test de dissolution *in vitro* peut être réalisé à l'aide de tout dispositif disponible dans le commerce. L'exemple 1 ci-après présente des conditions de mise en oeuvre pour la détermination de la vitesse de dissolution *in vitro* d'un co-micronisat selon l'invention. Brièvement, une quantité de co-micronisat représentant 30 mg de principe actif est disposé dans une gélule en gélatine. Cette gélule est ensuite placée dans 900 ml d'un milieu tamponné à pH gastrique, comprenant 0,1% de SDS, à 37±0,5 °C et soumis à une agitation de 50 tours par minute (rpm) (vitesse de rotation des pales du dispositif de dissolution). La dissolution du principe actif dans le milieu peut être suivie par spectrophotométrie, par exemple à la longueur d'onde d'absorbance maximale. Au sens de l'invention, un pH gastrique est typiquement un pH de 1 à 3.

« Au moins 55% du principe actif libéré en 45 minutes » englobe au moins 55%, au moins au moins 60%, au moins 65%, au moins 70%, au moins 75%, au moins 80% du principe actif est libéré en 45 minutes.

De préférence, au moins 60% du principe actif est libéré en 45 minutes.

Le produit de co-micronisat peut être, en outre, caractérisé en ce qu'au moins 45% du principe actif qu'il contient est libéré en 30 minutes lorsqu'il est soumis à un test de dissolution in vitro comme décrit précédemment.

La présente invention a également pour objet un procédé de préparation du co-micronisat ci-dessus décrit, comprenant les étapes consistant à :
a) fournir un principe actif choisi parmi le groupe constitué par l'ulipristal acétate, un métabolite de l'ulipristal acétate et leurs mélanges,
b) mélanger le principe actif de l'étape a) avec un excipient polymérique et
c) microniser le mélange obtenu à l'étape b),
étant entendu que le principe actif et l'excipient polymérique sont tels que définis précédemment.

Le principe actif fourni à l'étape a) peut être sous forme micronisée ou non-micronisée. Par ailleurs, le principe actif peut être amorphe ou cristallin. De manière préférée, le principe actif fournit à l'étape a) est sous forme cristalline.

Dans certains modes de réalisation, l'étape b) peut, de plus, comprendre le mélange du principe actif avec un excipient pharmaceutique additionnel, de préférence un surfactant. Ce mélange peut intervenir avant, simultanément ou après le mélange du principe actif avec l'excipient polymérique. L'excipient pharmaceutique additionnel peut être fourni sous forme micronisée ou non-micronisée.

L'étape c) de micronisation peut être réalisée à l'aide d'un système de micronisation disponible dans le commerce. Il peut s'agir, en particulier, d'un microniseur à jet d'air ou d'un microniseur à boules. L'homme du métier, grâce à ses connaissances générales et à la mise en oeuvre d'expériences de routine, saura déterminer les conditions de mise en oeuvre de l'étape c) afin d'obtenir un produit de co-micronisation présentant la granulométrie désirée. A titre d'exemple, lorsque l'étape c) est mise en oeuvre à l'aide d'un microniseur à jet d'air, l'homme du métier pourra faire varier le débit d'alimentation en poudre et la pression des jets d'air afin de moduler la granulométrie du co-micronisat final.

### Composition pharmaceutique selon l'invention

Le produit de co-micronisation est destiné principalement à une utilisation thérapeutique ou contraceptive. A cette fin, il peut être administré directement ou inséré dans un dispositif d'administration tel qu'un anneau vaginal, un patch, un stérilet ou un implant.

En général, le produit de co-micronisation selon l'invention est intégré dans une composition pharmaceutique de manière à faciliter son administration. Ainsi, un objet supplémentaire de la présente invention est une composition pharmaceutique comprenant un produit de co-micronisation tel que défini précédemment et au moins un excipient acceptable sur le plan pharmaceutique.

L'homme du métier saura choisir le ou les excipients à associer au produit de co-micronisation en fonction de la forme finale de la composition pharmaceutique, de la voie d'administration souhaitée et du profil de libération du principe actif recherché. A cette fin, l'homme du métier pourra se référer aux ouvrages de référence suivants : Remington: The Science and Practice of Pharmacy (Lippincott Williams & Wilkins; Twenty first Edition, 2005) et, Handbook of Pharmaceuticals Excipients, American Pharmaceutical Association (Pharmaceutical Press; 6th revised edition, 2009).

La composition pharmaceutique et le co-micronisat selon l'invention peuvent être administrés par toute voie, en particulier par voie orale, buccale, nasale, sublinguale, vaginale, intrautérine, rectale, transdermale ou par voie parentérale, par exemple par injection intraveineuse. Les voies d'administration préférées sont les voies buccale, orale, intrautérine et vaginale.

La composition pharmaceutique selon l'invention peut se présenter sous toute forme, par exemple sous la forme d'un comprimé, d'une poudre, d'une gélule, d'une pilule, d'un suppositoire, d'une ovule, d'une suspension, d'une solution aqueuse, alcoolique ou huileuse, d'un sirop, d'un gel, d'une pommade, d'une émulsion, d'un lyophilisat ou d'un film orodispersible. La voie d'administration et la forme galénique de la composition pharmaceutique pourront dépendre de l'effet thérapeutique ou contraceptif recherché.

Dans certains modes de réalisation, la composition pharmaceutique selon l'invention peut être intégrée dans un dispositif permettant l'administration prolongée du principe actif. La composition pharmaceutique pourra être, en particulier, intégrée dans un anneau vaginal, dans un stérilet, dans un patch, par exemple un patch transdermique ou muco-adhésif, ou dans un implant, par exemple un implant de type contraceptif. Pour des exemples d'anneaux vaginaux adaptés à la mise en oeuvre de l'invention, on pourra se référer à la demande WO2006/10097.

Dans des modes de réalisation supplémentaires, la composition pharmaceutique selon l'invention se présente sous forme solide. De préférence, la composition pharmaceutique selon l'invention est solide et destinée à une administration orale.

Dans certains modes de réalisation, la composition pharmaceutique selon l'invention est caractérisée en ce que l'excipient acceptable sur le plan pharmaceutique est choisi parmi le groupe constitué par un diluant, un liant, un agent d'écoulement, un lubrifiant, un agent désintégrant et leurs mélanges.

Un diluant au sens de la présente invention peut être un ou plusieurs composés capables de densifier le principe actif pour obtenir la masse désirée. Les diluants englobent les phosphates minéraux, les monosaccharides et les polyols tels que le xylitol, le sorbitol, le lactose, le galactose, le xylose ou le mannitol, les disaccharides tels que le saccharose, les oligosaccharides, les polysaccarides tels que la cellulose et ses dérivés, les amidons, et les mélanges.

Le diluant peut être sous forme anhydre ou hydraté.

A titre d'exemple, un diluant adapté selon l'invention peut être choisi parmi la cellulose microcristalline, le mannitol, le lactose et leurs mélanges.

Le liant peut être un ou plusieurs composés capables d'améliorer l'agrégation du principe actif avec le diluant. On peut citer, à titre d'exemple de liants, l'hydroxypropyl cellulose, l'hydroxypropylméthyl cellulose, la povidone (polyvinylpyrrolidone), les copolymères de N-vinyl-2-pyrrolidone et d'acétate de vinyle (copovidone) et leurs mélanges.

Le lubrifiant peut être un ou plusieurs composés capables d'empêcher les problèmes liés à la préparation des formes galéniques sèches, comme les problèmes de collage et/ou de grippage qui surviennent au niveau des machines lors de la compression ou du remplissage. Les lubrifiants préférés sont des acides gras ou des dérivés d'acides gras comme le stéarate de calcium, le monostéarate de glycéryle, le palmitostéarate de glycéryle, le stéarate de magnésium, le stéarate de zinc, ou l'acide stéarique, les polyalkylèneglycols, notamment le polyéthylèneglycol, le benzoate de sodium ou le talc. Les lubrifiants préférés selon l'invention sont les sels de stéarates et leurs mélanges. Un lubrifiant adapté est par exemple le stéarate de magnésium.

L'agent d'écoulement éventuellement employé selon invention peut être choisi parmi les composés qui contiennent du silicium, par exemple, le talc, la silice colloïdale anhydre ou de la silice précipitée.

Le désintégrant peut être utilisé pour améliorer la libération du principe actif. Il peut être choisi, par exemple, parmi la polyvinyl pyrrolidone réticulée (crospovidone), la carboxyméthyl cellulose réticulée (telle que la croscarmellose de sodium) ou non-réticulée, les amidons et leurs mélanges. Le désintégrant est de préférence choisi dans le groupe constitué par une croscarmellose sodique, une crospovidone et leurs mélanges.

Dans certains modes de réalisation, la composition selon l'invention comprend :
- 0,5% à 80% du produit de co-micronisation tel que défini précédemment,
- 15% à 95 % de diluant, de préférence choisi parmi le mannitol, le lactose, la cellulose microcristalline et leurs mélanges, et
- 0% à 5% de lubrifiant, de préférence un stéarate tel que le stéarate de magnésium,
les pourcentages étant exprimés en poids par rapport au poids total de la composition.

La composition selon l'invention peut être, en outre, caractérisée en ce qu'elle comprend de 0% à 20% en poids d'un agent liant, 0% à 10% d'agent désintégrant, et de 0% à 5% en poids d'un agent d'écoulement.

Il est à noter que le pourcentage massique d'agent désintégrant de la composition pharmaceutique selon l'invention ne prend pas en compte la crospovidone éventuellement contenue dans le co-micronisat. De même, le pourcentage massique d'agent liant de la composition pharmaceutique selon l'invention ne comprend pas la povidone éventuellement contenue dans le co-micronisat.

Dans d'autres modes de réalisation, la composition pharmaceutique selon l'invention comprend de :
- 1% à 60% en poids de produit de co-micronisation tel que précédemment décrit,
- 30% à 95% en poids de diluant
- 0% à 10% en poids d'agent désintégrant
- 0% à 10% en poids d'un agent liant,
- 0% à 5% en poids d'un agent d'écoulement et
- 0,1% à 2% d'un lubrifiant.
les pourcentages étant exprimés en poids par rapport au poids total de la composition.

La composition pharmaceutique selon l'invention peut comprendre, en outre, un ou plusieurs excipients additionnels aux excipients précédemment cités. Le ou les excipients additionnels peuvent être choisis parmi le groupe constitué par les surfactants tels que le SDS, les agents d'enrobage tels que les agents d'enrobage à base d'alcool polyvynilique ou d'hydroxypropylmethylcellulose, les pigments tels que l'oxyde d'aluminium ou l'oxyde de fer, les arômes, les agents mouillants, les cires, les agents dispersants, les stabilisants et les conservateurs.

La composition pharmaceutique selon l'invention pourra être préparée selon l'une quelconque des méthodes couramment utilisées en galénique. Ces méthodes comprennent typiquement le mélange du produit de co-micronisation selon l'invention avec un ou plusieurs excipients puis la mise en forme du mélange obtenu. A titre d'exemple, lorsqu'elle se présente sous la forme d'un comprimé, la composition pharmaceutique selon l'invention peut être préparée, par compression directe ou par compression après granulation par voie sèche ou humide.

Le produit de co-micronisation présent dans la composition pharmaceutique selon l'invention peut avoir l'une quelconque des caractéristiques décrites dans la présente description. En particulier, le produit de co-micronisation de la composition pharmaceutique présente une ou plusieurs (1, 2, 3, 4, 5, 6, 7 ou 8) des caractéristiques suivantes :
i. le principe actif est l'UPA
ii. l'excipient polymérique est choisi parmi le groupe constitué par les povidones, les crospovidones et leurs mélanges
iii. le « rapport massique principe actif/excipient polymérique » est compris dans une gamme allant de 0,5 à 4,
iv. il comprend en outre un surfactant, de préférence le SDS
v. lorsque le surfactant est présent, le « rapport massique principe actif/surfactant » est de 0,5 à 4,
vi. la d50 du produit de co-micronisation est inférieure à 20 µm, de préférence inférieure à 15 µm,
vii. la d90 du produit de comicronisation est inférieure à 50 µm, de préférence inférieure à 40 µm, et
viii. au moins 55% du principe actif que le produit de co-micronisation contient est libéré en 45 minutes lorsque ledit produit de co-micronisation est soumis à un test de dissolution *in vitro,* de préférence dans les conditions suivantes :
   - dispositif: dispositif de dissolution à pales,
   - échantillon : gélule en gélatine dure contenant une quantité de co-micronisat correspondant à 30 mg de principe actif,
   - milieu de dissolution : 900 ml d'une solution aqueuse tamponnée à pH gastrique comprenant 0,1% de SDS,
   - température : 37±0,5 °C, et
   - vitesse de rotation des pales : 50 tours par minute (rpm)

Comme mentionné précédemment, la composition selon l'invention peut se présenter sous la forme d'une poudre, d'une granule, d'un comprimé pelliculé ou non pelliculé, ou d'une gélule, et est de préférence destinée à une administration orale. Dans certains modes de réalisation, la composition pharmaceutique selon l'invention se présente sous la forme d'un comprimé non-pelliculé destiné à une administration orale.

La composition selon l'invention peut être une composition pharmaceutique à libération contrôlée, immédiate, prolongée ou retardée. De préférence, la composition selon l'invention est une composition à libération immédiate.

On entend par une « composition à libération immédiate », une composition pharmaceutique caractérisée en ce qu'au moins 75% du principe actif initialement contenu dans une unité de dose de la composition pharmaceutique est libéré en 45 minutes lorsque ladite unité de dose est soumise à un test de dissolution *in vitro,* par exemple selon la Pharmacopée Européenne §2.9.3, et de préférence, dans les conditions suivantes :
- dispositif de dissolution à pales,
- milieu de dissolution : solution aqueuse tamponnée à pH gastrique contenant 0,1% de SDS
- température : 37±0.5°C, et
- vitesse de rotation : 50 rpm.

Le volume du milieu de dissolution dépend de la quantité de principe actif contenue dans l'unité de dose. Pour une unité de dose comprenant 5 mg de principe actif, on utilise 500 ml de milieu de dissolution. Pour une unité de dose comprenant 30 mg de principe actif, on utilise 900 ml de milieu de dissolution.

De manière générale, la composition pharmaceutique comprend de 1 mg à 100 mg de principe actif par unité de dose, de préférence de 1 mg à 40 mg, voire de 2 mg à 30 mg, de principe actif par unité de dose. La dose de principe actif est fonction de l'effet thérapeutique ou contraceptif et du schéma d'administration recherchés. Par exemple pour certaines applications, la quantité d'UPA par unité de dose peut être comprise dans une gamme allant de 1 mg à 5 mg.

En contraception d'urgence, le principe actif peut être présent à hauteur de 20 mg à 40 mg par unité de dose.

En contraception régulière, le principe actif peut être présent à hauteur de 2 mg à 5 mg par unité de dose.

Pour des utilisations thérapeutiques tels que le traitement des fibromes utérins, le principe actif peut être présent à hauteur de 3 mg à 15 mg par unité de dose.

La dose en principe actif et le schéma d'administration pourra également dépendre des paramètres personnels du patient, en particulier de son poids, son âge, son sexe, son état de santé général, son régime alimentaire, des pathologies dont il souffre...

Enfin, la composition pharmaceutique selon l'invention peut comprendre un principe actif supplémentaire. Cet actif supplémentaire peut exercer une action distincte de celle de l'UPA ou de ses métabolites. Il peut également renforcer l'effet thérapeutique de l'UPA ou de ses métabolites.

### Utilisations thérapeutiques ou contraceptiques du co-micronisat et de la composition pharmaceutique selon la présente invention

Dans un aspect supplémentaire, la présente invention a également pour objet un produit de co-micronisation ou une composition pharmaceutique tels que décrits précédemment pour une utilisation en tant que médicament. Le produit de co-micronisation ou la composition selon l'invention sont particulièrement adaptés pour une utilisation en tant que contraceptif régulier ou contraceptif d'urgence. Ils peuvent être également utilisés pour le traitement ou la prévention de troubles hormonaux, gynécologiques ou endocriniens tels que la maladie de Cushing. La composition ou le produit de co-micronisation selon l'invention peuvent être utilisés, en particulier, dans le traitement ou la prévention d'un trouble gynécologique, de préférence affectant l'utérus, y compris les troubles gynécologiques bénins. Les troubles gynécologiques englobent, sans y être limités, les fibromes utérins et ses symptômes, l'adénomyose, l'endométriose, les douleurs associées à la dislocation de l'endomètre, les saignements utérins excessifs.

Un objet supplémentaire de l'invention est l'utilisation du produit de co-micronisation selon l'invention pour la préparation d'un contraceptif ou pour la préparation d'un médicament destiné au traitement ou à la prévention de l'une quelconque des pathologies ci-dessus citées.

L'invention a également pour objet une méthode de contraception comprenant l'administration à une patiente d'une dose contraceptive du produit de co-micronisation ou de la composition pharmaceutique selon l'invention.

On entend par « méthode de contraception » une méthode permettant d'éviter la survenue d'une grossesse chez une patiente en âge de procréer.

Dans le cas d'espèce, il peut s'agir d'une méthode de contraception d'urgence. Dans ce cas, une dose unique est de préférence administrée à la patiente dans un intervalle de temps adéquate après le rapport non ou mal protégé, en général dans les 120 h suivant le rapport non ou mal protégé. La méthode de contraception peut être également une méthode de contraception régulière, dans laquelle la composition ou le produit de co-micronisation sont administrés de manière chronique et cyclique à la patiente ou de manière continue à l'aide d'un dispositif tel qu'un implant ou un anneau vaginal. A titre alternatif, la méthode de contraception peut être une méthode de contraception dite « à la demande » telle que décrite dans la demande internationale WO2010/119029. Enfin, l'invention a également pour objet une méthode de traitement d'une maladie ou d'un trouble chez un patient comprenant l'administration d'une dose thérapeutiquement efficace du produit de co-micronisation ou de la composition pharmaceutique selon l'invention au patient, de préférence de sexe féminin. La méthode thérapeutique selon l'invention concerne, de préférence, l'un quelconque des maladies ou troubles cités ci-dessus. Il va de soi que pour la mise en oeuvre des méthodes et utilisations ci-dessus décrites, le produit de co-micronisation et la composition pharmaceutique selon l'invention peuvent comprendre une ou plusieurs des caractéristiques détaillées dans la présente description.

Les exemples ci-après ont pour but d'illustrer plus pleinement l'invention sans pour autant en limiter la portée.

### EXEMPLES

### Exemple 1 : Préparation de co-micronisats et criblage d'excipients

### 1. Matériel et méthode

### ▪ Préparation des co-micronisats

Les produits de co-micronisation (ci-après « co-micronisats ») d'ulipristal acétate ont été préparés selon la méthode suivante : L'ulipristal acétate et l'excipient de co-micronisation à tester ont été mélangés selon le rapport massique désiré dans un mortier et triturés jusqu'à l'obtention d'un mélange homogène. Le mélange obtenu a été micronisé à l'aide d'un broyeur-homogénéiseur à billes de manière à obtenir la granulométrie souhaitée.

### ▪ Dissolution in vitro des co-micronisats d'UPA

Pour chaque co-micronisat obtenu, on a préparé des gélules en gélatine dure contenant une quantité de co-micronisat correspondant à 30 mg d'UPA par gélule. Les études de dissolution *in vitro* de l'UPA en co-micronisat ont été réalisées à partir de ces gélules selon la méthode décrite dans la Pharmacopée Européenne au §2.9.3 en utilisant un dispositif de dissolution à pales.

Pour chaque co-micronisat, une gélule contenant ledit co-micronisat a été placée dans un bol du dispositif de dissolution contenant 900 ml d'un milieu de dissolution. Le milieu de dissolution est une solution aqueuse tamponnée à pH gastrique et comprenant 0,1% en poids de SDS. Les conditions de mise en oeuvre des dissolutions *in vitro* sont les suivantes :
- Vitesse de rotation des pales : 50 tours par minute (rpm)
- Température : 37°C ± 0,5°C

La dissolution de l'UPA a été suivie par spectrophotométrie.

A titre d'expérience témoin, on a utilisé une gélule en gélatine contenant 30 mg d'ulipristal acétate micronisé seul (c'est-à-dire d'UPA micronisé en l'absence de tout excipient de co-micronisation). Pour chaque co-micronisat, l'expérience de dissolution a été reproduite 3 fois.

### 2. Résultats

### ▪ Série d'expériences 1 : Criblage d'excipient de co-micronisation

Le tableau 1 ci-dessous et la Figure 1 montrent les résultats de dissolution obtenus pour chaque co-micronisat préparé. Les pourcentages de dissolution sont exprimés par rapport à la quantité initiale d'UPA contenue dans chaque gélule.

**Tableau 1 : Résultats des essais de dissolution in vitro pour les co-micromisats d'UPA/excipient préparés. Rapport massique UPA/excipient 7/3. Expérience témoin : UPA micronisé seul.**

| | Pourcentages d'UPA libéré exprimés par rapport à la quantité initiale d'UPA contenue par gélule. (Valeurs moyennes sur 3 expériences) | | | | | |
|---|---|---|---|---|---|---|
| Temps (min) | UPA micronisée | Povidone (Plasdone® K29-32) | Crospovidone (Polyplasdone® XL10) | Kollicoat® IR | Acide citrique monohydrate | Acide fumarique |
| 0 | 0,00 | 0,00 | 0,00 | 0,00 | 0,00 | 0,00 |
| 1 | 0,00 | 0,40 | 0,00 | 0,20 | 0,10 | 1,10 |
| 5 | 5,50 | 1,00 | 0,50 | 0,50 | 0,50 | 1,20 |
| 7,5 | 15,00 | 1,70 | 2,60 | 0,70 | 0,60 | 1,80 |
| 10 | 22,30 | 4,10 | 6,80 | 0,90 | 0,80 | 2,10 |
| 15 | 30,00 | 14,50 | 17,10 | 1,10 | 1,10 | 2,30 |
| 20 | 34,30 | 30,60 | 34,80 | 1,40 | 1,60 | 2,90 |
| 30 | 39,50 | 52,50 | 69,90 | 2,50 | 2,60 | 5,70 |
| 45 | 47,50 | 63,20 | 88,90 | 5,30 | 4,70 | 11,00 |
| 60 | 53,80 | 69,10 | 92,60 | 8,80 | 7,10 | 16,40 |

On précise que le Kollicoat IR® est un copolymère greffé de polyéthylène glycol et d'alcool polyvinylique..

Ces résultats montrent que la co-micronisation de l'ulipristal acétate avec une crospovidone ou une povidone permet d'améliorer de manière très significative la quantité finale (à t = 60 min) d'UPA libéré et donc la vitesse globale de dissolution. De manière remarquable, le pourcentage d'ulipristal acétate libéré dans le milieu à t = 30 min est d'environ 52% pour une gélule comprenant le co-micronisat UPA/povidone et d'environ 69% pour une gélule comprenant le co-micronisat UPA/crospovidone alors qu'il n'est que d'environ 39% pour une gélule contenant de l'ulipristal acétate micronisé.

Le co-micronisat d'UPA et de Kollicoat IR® présente une vitesse de libération de l'UPA très inférieure à celle observée pour l'UPA micronisé puisqu'au bout de 60 min, moins de 10% de l'UPA contenu initialement dans les co-micronisats est libéré. La solubilité de l'ulipristal acétate étant pH-dépendante (voir tableau 2 ci-dessous), il était attendu que la co-micronisation de l'ulipristal d'acétate avec un excipient acide - tel que l'acide citrique ou l'acide fumarique - permette d'améliorer la dissolution de l'ulipristal acétate en diminuant le pH dans l'environnement proche de la forme de dosage et donc en augmentant localement sa solubilité.

**Tableau 2 : solubilité de l'UPA en fonction du pH**

| pH | Solubilité UPA (g/L) |
|---|---|
| 1.2 | 22,7 |
| 4.5 | 0,039 |
| 6.8 | 0,005 |

De manière surprenante, la co-micronisation de l'UPA avec un acide organique entraîne une diminution nette de la vitesse et du taux final (à t = 60 min) de dissolution de l'UPA.

### Conclusion

La co-micronisation en présence d'un polymère à base de N-vinylpyrrolidone tel qu'une povidone ou une crospovidone a permis d'améliorer le profil de dissolution de l'UPA *in vitro* par rapport à l'UPA sous forme micronisée. En revanche, contrairement à ce que l'on aurait pu attendre, les autres excipients de co-micronisation testés dans cet exemple ont eu un impact nettement négatif sur la libération de l'UPA.

### ▪ Série d'expérience 2 : effet de l'ajout d'un surfactant

Les co-micronisats suivants ont été préparés :
- Co-micronisat UPA/crospovidone 7/3 et
- Co-micronisat UPA/crospovidone/SDS 5/2/3

On a également préparé une matrice de principe actif en mélangeant un co-micronisat UPA/crospovidone 5/2 avec du SDS afin d'obtenir un mélange UPA/crospovidone/SDS 5/2/3. Cette matrice est appelée ci-après UPA/crospovidone/SDS externe.

On a utilisé en tant qu'expérience témoin une poudre d'UPA micronisée.

Ces différentes matrices de principe actif ont été soumises à un test de dissolution *in vitro* tel que décrit ci-avant dans la partie « Matériel et méthodes ».

Les dissolutions *in vitro* obtenues sont illustrées à la Figure 2 et présentées dans le Tableau 3 ci-après.

**Tableau 3 : Résultats de dissolution in vitro**

| | Pourcentages d'UPA libéré exprimés par rapport à la quantité initiale d'UPA contenue par gélule. (Valeurs moyennes sur 3 expériences) | | | |
|---|---|---|---|---|
| Temps (min) | UPA micronisée (comparatif) | UPA/crospovidone 7/3 (invention) | UPA/crospovidone/SDS (5/2/3) (invention) | UPA/crospovidone/SDS externe (5/2/3) (invention et comparatif) |
| 0 | 0,00 | 0,00 | 0,00 | 0,00 |
| 1 | 0,00 | 0,00 | 0,40 | 0,00 |
| 5 | 5,50 | 0,50 | 7,10 | 2,80 |
| 7,5 | 15,00 | 2,60 | 18,00 | 10,30 |
| 10 | 22,30 | 6,80 | 28,00 | 25,80 |
| 15 | 30,00 | 17,10 | 58,30 | 40,70 |
| 20 | 34,30 | 34,80 | 67,60 | 48,10 |
| 30 | 39,50 | 69,90 | 80,70 | 58,70 |
| 45 | 47,50 | 88,90 | 85,40 | 66,10 |
| 60 | 53,80 | 92,60 | 86,80 | 70,40 |

On observe une amélioration de la dissolution in vitro pour tous les co-micronisats testés. L'amélioration est moins marquée pour la matrice UPA/Crospovidone/SDS externe, ce qui illustre l'effet spécifique de la co-micronisation (par rapport à un mélange physique).

### Exemple 2 : Compositions pharmaceutiques intégrant le co-micronisat selon l'invention

Les tableaux 4 et 5 ci-après présentent des exemples de composition pharmaceutique selon l'invention. Ces compositions pharmaceutiques peuvent être préparées par compression directe d'un mélange comprenant le co-micronisat et les différents excipients.

**Tableau 4 : Exemple d'une composition selon l'invention à 5 mg d'UPA**

| Ingrédients | Fonction | % en poids | mg/comprimé |
|---|---|---|---|
| Co-micronisat UPA/crospovidone 7/3 | Matrice de principe actif | 4,7 | 7,1 (soit 5 mg d'UPA) |
| Cellulose microcristalline | Diluant | 60,8 | 91,2 |
| Mannitol | Diluant | 29,0 | 43,5 |
| Crospovidone | Désintégrant | 4,9 | 7,4 |
| Stéarate de magnésium | Lubrifiant | 0,5 | 0,8 |
| Total | | | 150 |

Cette composition peut être utilisée, par exemple, pour le traitement des fibromes utérins.

**Tableau 5 : Exemple d'une composition selon l'invention à 30 mg d'UPA**

| Ingrédients | Fonction | % en poids | mg/comprimé |
|---|---|---|---|
| Co-micronisat UPA/crospovidone 7/3 | Matrice de principe actif | 28,4 | 42,6 (soit 30 mg d'UPA) |
| Cellulose microcristalline | Diluant | 37,1 | 55,7 |
| Mannitol | Diluant | 29,0 | 43,5 |
| Crospovidone | Désintégrant | 4,9 | 7,4 |
| Stéarate de magnésium | Lubrifiant | 0,5 | 0,8 |
| Total | | | 150 |

Cette composition peut être utilisée, par exemple, en contraception d'urgence.

## Revendications

1. Produit de co-micronisation comprenant :
- un principe actif choisi parmi le groupe constitué par les modulateurs sélectifs des récepteurs à la progestérone, leurs métabolites et leurs mélanges, et
- un excipient polymérique choisi parmi le groupe constitué par les polymères à base de N-vinyl-2-pyrrolidone et leurs mélanges.

2. Produit de co-micronisation selon la revendication 1 **caractérisé en ce que** le principe actif est choisi parmi le groupe constitué par la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, l'ulipristal acétate et leurs mélanges.

3. Produit de co-micronisation selon l'une quelconque des revendications 1 à 2 **caractérisé en ce que** le rapport massique « principe actif / excipient polymérique » est compris dans une gamme allant de 0,1 à 10, de préférence de 0,5 à 4.

4. Produit de co-micronisation selon l'une quelconque des revendications 1 à 3 **caractérisé en ce que** :
▪ le principe actif est l'ulipristal acétate et
▪ l'excipient polymérique est choisi parmi le groupe constitué par une polyvinylpyrrolidone non-réticulée, une polyvinylpyrrolidone réticulée et leurs mélanges.

5. Produit de co-micronisation selon l'une quelconque des revendications 1 à 4 **caractérisé en ce qu'**il comprend, en outre, un surfactant solide, de préférence le dodécylsulfate de sodium.

6. Produit de co-micronisation selon l'une quelconque des revendications 1 à 5 ayant:
- une d50 inférieure à 20 µm, de préférence inférieure à 15 µm, et/ou
- une d90 inférieure à 50 µm, de préférence inférieure à 40 µm.

7. Procédé de préparation d'un produit de co-micronisation tel que défini dans l'une quelconque des revendications 1 à 6, comprenant les étapes consistant à :
a) fournir un principe actif tel que défini dans l'une quelconque des revendications 1 ou 2 ;
b) mélanger le principe actif de l'étape a) avec un excipient polymérique tel que défini dans la revendication 1 et, en option, avec un surfactant solide, de préférence le dodécylsulfate de sodium ; et
c) co-microniser le mélange obtenu à l'étape b).

8. Composition pharmaceutique comprenant un produit de co-micronisation tel que défini dans l'une des revendications 1 à 6 et un excipient acceptable sur le plan pharmaceutique

9. Composition pharmaceutique selon la revendication 8 **caractérisée en ce que** l'excipient acceptable sur le plan pharmaceutique est choisi parmi le groupe constitué par un diluant, un liant, un agent d'écoulement, un lubrifiant, un agent désintégrant et leurs mélanges.

10. Composition pharmaceutique selon l'une des revendications 8 et 9 comprenant de :
- 0,5% à 80% de produit de co-micronisation,
- 15% à 95 % de diluant, et
- 0% à 5% de lubrifiant,
les pourcentages étant exprimés en poids par rapport au poids total de la composition.

11. Composition pharmaceutique selon l'une des revendications 8 à 10 **caractérisée en ce qu'**elle comprend de 1 mg à 100 mg, de préférence de 1 mg à 40 mg de principe actif par unité de dose.

12. Composition pharmaceutique selon l'une des revendications 8 à 11 **caractérisée en ce qu'**elle est adaptée à une administration par voie orale.

13. Composition pharmaceutique selon l'une des revendications 8 à 12 **caractérisée en ce qu'**elle est sous la forme d'une poudre, d'une granule, d'un comprimé pelliculé ou non pelliculé, ou d'une gélule.

14. Produit de co-micronisation selon l'une quelconque des revendications 1 à 6 ou composition pharmaceutique selon l'une des revendications 8 à 13 pour une utilisation en tant que contraceptif.

15. Produit de co-micronisation selon l'une quelconque des revendications 1 à 6 ou composition pharmaceutique selon l'une des revendications 8 à 13 pour une utilisation dans le traitement ou la prévention d'un trouble gynécologique, de préférence affectant l'utérus.

## Patentansprüche

1. Co-Mikronisierungsprodukt, umfassend:
- einen Wirkstoff, ausgewählt aus der Gruppe, bestehend aus selektiven Progesteronrezeptormodulatoren, Metaboliten davon und Gemischen davon, und
- einen polymeren Arzneimittelträger, ausgewählt aus der Gruppe, bestehend aus Polymeren auf Basis von N-Vinyl-2-pyrrolidon und Gemischen davon.

2. Co-Mikronisierungsprodukt gemäß Anspruch 1, **dadurch gekennzeichnet, dass** der Wirkstoff aus der Gruppe ausgewählt ist, bestehend aus 17α-Acetoxy-11β-[4-N-methylamino-phenyl]-19-norpregna-4,9-dien-3,20-dion, 17α-Acetoxy-11β-[4-amino-phenyl]-19-norpregna-4,9-dien-3,20-dion, Ulipristalacetat und Gemischen davon.

3. Co-Mikronisierungsprodukt gemäß einem der Ansprüche 1 bis 2, **dadurch gekennzeichnet, dass** das Massenverhältnis von Wirkstoff zu polymerem Arzneimittelträger einen Bereich von 0,1 bis 10, vorzugsweise 0,5 bis 4, umfasst.

4. Co-Mikronisierungsprodukt gemäß einem der Ansprüche 1 bis 3, **dadurch gekennzeichnet, dass**:
▪ der Wirkstoff Ulipristalacetat ist, und
▪ der polymere Arzneimittelträger aus der Gruppe ausgewählt ist, bestehend aus einem nicht vernetzten Polyvinylpyrrolidon, einem vernetzten Polyvinylpyrrolidon und Gemischen davon.

5. Co-Mikronisierungsprodukt gemäß einem der Ansprüche 1 bis 4, **dadurch gekennzeichnet, dass** es außerdem eine feste oberflächenaktive Substanz, vorzugsweise Natriumdodecylsulfat, umfasst.

6. Co-Mikronisierungsprodukt gemäß einem der Ansprüche 1 bis 5 mit:
- einem d50 kleiner als 20 µm, vorzugsweise kleiner als 15 µm, und/oder
- einem d90 kleiner als 50 µm, vorzugsweise kleiner als 40 µm.

7. Verfahren zur Herstellung eines Co-Mikronisierungsprodukts wie in einem der Ansprüche 1 bis 6 definiert, umfassend die Schritte, bestehend aus:
a) Bereitstellen eines Wirkstoffs wie in einem der Ansprüche 1 oder 2 definiert;
b) Mischen des Wirkstoffs von Schritt a) mit einem polymeren Arzneimittelträger wie in Anspruch 1 definiert und gegebenenfalls mit einer festen oberflächenaktiven Substanz, vorzugsweise Natriumdodecylsulfat; und
c) Co-Mikronisieren des in Schritt b) erhaltenen Gemisches.

8. Pharmazeutische Zusammensetzung, umfassend ein Co-Mikronisierungsprodukt wie in einem der Ansprüche 1 bis 6 definiert und einen pharmazeutisch verträglichen Arzneimittelträger.

9. Pharmazeutische Zusammensetzung gemäß Anspruch 8, **dadurch gekennzeichnet, dass** der pharmazeutisch verträgliche Arzneimittelträger aus der Gruppe ausgewählt ist, bestehend aus einem Verdünnungsmittel, einem Bindemittel, einem Fließmittel, einem Gleitmittel, einem Sprengmittel und Gemischen davon.

10. Pharmazeutische Zusammensetzung gemäß einem der Ansprüche 8 und 9, umfassend:
- 0,5% bis 80% Co-Mikronisierungsprodukt,
- 15% bis 95% Verdünnungsmittel, und
- 0% bis 5% Gleitmittel,
wobei die prozentualen Anteile als Gewichtsprozente bezogen auf das Gesamtgewicht der Zusammensetzung ausgedrückt sind.

11. Pharmazeutische Zusammensetzung gemäß einem der Ansprüche 8 bis 10, **dadurch gekennzeichnet, dass** sie 1 mg bis 100 mg, vorzugsweise 1 mg bis 40 mg, Wirkstoff pro Dosiseinheit umfasst.

12. Pharmazeutische Zusammensetzung gemäß einem der Ansprüche 8 bis 11, **dadurch gekennzeichnet, dass** sie für eine orale Verabreichung geeignet ist.

13. Pharmazeutische Zusammensetzung gemäß einem der Ansprüche 8 bis 12, **dadurch gekennzeichnet, dass** sie in Form eines Pulvers, eines Granulats, einer Filmtablette oder einer filmlosen Tablette oder einer Kapsel vorliegt.

14. Co-Mikronisierungsprodukt gemäß einem der Ansprüche 1 bis 6 oder pharmazeutische Zusammensetzung gemäß einem der Ansprüche 8 bis 13 für eine Verwendung als Kontrazeptivum.

15. Co-Mikronisierungsprodukt gemäß einem der Ansprüche 1 bis 6 oder pharmazeutische Zusammensetzung gemäß einem der Ansprüche 8 bis 13 für eine Verwendung in der Behandlung oder Prävention einer bevorzugt den Uterus betreffenden gynäkologischen Störung.

## Claims

1. Co-micronization product comprising:
- an active ingredient selected from the group consisting of selective progesterone receptor modulators, metabolites thereof and mixtures thereof, and
- a polymeric excipient selected from the group consisting of polymers based on N-vinyl-2-pyrrolidone and mixtures thereof.

2. Co-micronization product according to Claim 1, **characterized in that** the active ingredient is selected from the group consisting of 17α-acetoxy-11β-(4-N-methylaminophenyl)-19-norpregna-4,9-diene-3,20-dione, 17α-acetoxy-11β-(4-aminophenyl)-19-norpregna-4,9-diene-3,20-dione, ulipristal acetate and mixtures thereof.

3. Co-micronization product according to any one of Claims 1 and 2, **characterized in that** the "active ingredient/polymeric excipient" weight ratio is in a range from 0.1 to 10, preferably from 0.5 to 4.

4. Co-micronization product according to any one of Claims 1 to 3, **characterized in that**:
▪ the active ingredient is ulipristal acetate and
▪ the polymeric excipient is selected from the group consisting of a non-crosslinked polyvinylpyrrolidone, a crosslinked polyvinylpyrrolidone and mixtures thereof.

5. Co-micronization product according to any one of Claims 1 to 4, **characterized in that** it also comprises a solid surfactant, preferably sodium dodecyl sulfate.

6. Co-micronization product according to any one of Claims 1 to 5, having:
- a d50 of less than 20 µm, preferably less than 15 µm, and/or
- a d90 of less than 50 µm, preferably less than 40 µm.

7. Method for preparing a co-micronization product as defined in any one of Claims 1 to 6, comprising the steps consisting in:
a) providing an active ingredient as defined in any one of Claims 1 or 2;
b) mixing the active ingredient of step a) with a polymeric excipient as defined in Claim 1 and, optionally, with a solid surfactant, preferably sodium dodecyl sulfate; and
c) co-micronizing the mixture obtained in step b).

8. Pharmaceutical composition comprising a co-micronization product as defined in one of Claims 1 to 6 and a pharmaceutically acceptable excipient.

9. Pharmaceutical composition according to Claim 8, **characterized in that** the pharmaceutically acceptable excipient is selected from the group consisting of a diluent, a binder, a flow agent, a lubricant, a disintegrant and mixtures thereof.

10. Pharmaceutical composition according to either of Claims 8 and 9, comprising from:
- 0.5% to 80% of co-micronization product,
- 15% to 95% of diluent, and
- 0% to 5% of lubricant,
the percentages being expressed by weight as compared to the total weight of the composition.

11. Pharmaceutical composition according to one of Claims 8 to 10, **characterized in that** it comprises from 1 mg to 100 mg, preferably from 1 mg to 40 mg, of active ingredient per dose unit.

12. Pharmaceutical composition according to one of Claims 8 to 11, **characterized in that** it is suitable for oral administration.

13. Pharmaceutical composition according to one of Claims 8 to 12, **characterized in that** it is in the form of a powder, a granule, a film-coated or uncoated tablet, or a capsule.

14. Co-micronization product according to any one of Claims 1 to 6 or pharmaceutical composition according to one of Claims 8 to 13, for use as a contraceptive.

15. Co-micronization product according to any one of Claims 1 to 6 or pharmaceutical composition according to one of Claims 8 to 13, for use in the treatment or the prevention of a gynaecological disorder, preferably affecting the uterus.
